# EUROPEAN PATENT APPLICATION

(11) **EP 0 730 868 A1**
(43) Date of publication of application: **11.09.1996**
(21) Application number: 96650003.5
(22) Date of filing: 19.02.1996
(51) Int. Cl.: A61K 38/40, A23L 1/305

(54) **Formulated medicines for treatment of oral inflammation, and processed foods to treat and prevent stomatitis**

(30) Priority: 17.02.1995 JP 67158/95
(71) Applicant: Satoh, Tamotsu, Morioka-City, Iwate Prefecture (JP)
(72) Inventor: Satoh, Tamotsu, Morioka-City, Iwate Prefecture (JP); Satoh, Reeko, Morioka-City, Iwate Prefecture (JP); Naitoh, Yoshihisa, Morioka-City, Iwate Prefecture (JP)
(74) Representative: Gates, Marie Christina Esther

(57) **Abstract**

When the lactoferrin family protein is either orally administered, or topically applied to animal having oral inflammation such as aphthous stomatitis and gingivitis, the treatments much accelerate a recovery from the inflammation, thus, improve both (1) the efficacy of medicines such as anticancer chemotherapies, and (2) the quality of life in the patients with cancer, autoimmune diseases and gingivitis.

## Description

### Purpose of this invention:

The present invention relates to (A) formulated medicines or processed foods containing protein class lactoferrin family as active ingredient for treatment and prevention of aphthous stomatitis, (1) of cancer patients chronically received anticancer chemotherapies, (2) of patients with autoimmune disease, and (3) of patients with periodontal diseases in dentistry, (B) the method of treating and/or preventing aphthous stomatitis by using either the medicines or the foods or both which contain lactoferrin.

### Detailed Description of the Invention

### Background and Introduction:

Stomatitis is an inflammatory disease that occurs on oral cavity, tongue, gingivae and lips of patients with cancer and autoimmune diseases, or under malnutrition condition, or infants having acute epidemics. Recently, an attention is focused on treatment and prevention of aphthous stomatitis in cancer patients, because when they chronically receive cancer chemotherapies, approximate 95% of them are severely suffered from the stomatitis that much deteriorates the quality of life (QOL) of these patients. Aphthous stomatitis is a painful inflammation for the patients, but the disease is not fetal and had been regarded important, so that remedies are only symptomatic and can little improve the QOL. Therefore, new efficacious remedies against the stomatitis is longing and becomes one of the major targets for medical investigation.

Stomatitis is divided into two categories; the one is catarrhal stomatitis in infants, and the other is aphthous stomatitis. The former occurs in infants invaded by epidemics such as measles and scarlet fever. When catarrhal stomatitis occurs, oral mucosa becomes flushed, covered with thin white film and are apt to bleed from the mucosa. However, they are rapidly recovered from the illness because of their brisk restoration capacity. Therefore, it is not serious problem for infant health.

The most common and troublesome is an aphthous stomatitis that is either induced by, or complicated with the infection with herpes simplex virus, or by other agents including cancer chemotherapies, or both with autoimmune and periodontal diseases. For example, when the virus infects to unimmunised host, its proliferation develops fever 1-2 days later, and then, oral cavity becomes painful, and the patient slavers with bad breath accompanying with loss of appetite. Small blisters appear on oral mucosa and soon after bursting, they become circular ulcers of 1-2 mm diameter covered with pale yellow film. These aphthae are apt to appear on tongue and gingivae followed by swelling maxillary lymph node. The patients usually recover from fever 7 days later. However, troublesome is that they are often suffered from recurrence of the disease soon after recovery. This is a typical natural history of aphthous stomatitis caused by HSV infection.

Another causative agents of aphthous stomatitis are anticancer chemotherapies. Cancer is highly consumptive disease, and it is necessary for them to take enough meal for compensating the consumption. However, as mentioned above, anticancer chemotherapies induces the stomatitis at frequency higher than 95% in the patients. Consequently, they much decrease appetite due to the oral pain leading to body weight loss. Once the body weight begins to decrease in the patients due to poor appetite, any treatment no longer improves their condition. Moreover, the stomatitis is a highly recurrent disease, and even if the discontinuation cures it, it will soon recur by resuming the administration. Therefore, the stomatitis is one of the major obstacles to lower the efficacy of anticancer chemotherapies and effective therapies against the stomatitis are waiting for improve beneficial effect of anticancer chemotherapies.

Also, the stomatitis is one of the big problems in dentistry as well as autoimmune diseases, and new breakthrough is required to solve this problem. Because stomatitis frequently complicates with periodontal diseases such as gingivitis and periodontitis and it is difficult to treat such patients and the treatment should be waiting until the acute inflammation has been ceased. The patients with Bechet disease and systemic lupus erythematosus (SLE) are also suffered from the stomatitis; especially, the complication rate exceeds 98% in the case of Bechet disease. All of these instances indicate that aphthous stomatitis is the disease frequently encountered in the patients of cancer, and both periodontal and autoimmune diseases. Although it greatly deteriorates the QOL of these patients, there are no adequate means for the treatment, since it is not fetal.

### The problem to be solved by the present invention:

The inventors have been investigated the treatment methods of aphthous stomatitis in human and pets. During this investigation, the inventors found that 3 prerequisites should be needed for curative therapy of the stomatitis; that is, (1) an analgesic action relieving from pain, (2) local immunopotentiating action for clearing up mix-infected opportunistic pathogens, especially, in the case of periodontal diseases, (3) repairing action that accelerates the healing of aphthae and ulcers.

### The means to solve the problem:

The formulated medicine presented by this invention is composed of the protein class lactoferrin as an active ingredient. The protein can be recovered from the milk of humans, cows, goats, sheep, and the egg-white, and the activity is independent of the degree of iron saturation. Also, the human lactoferrin produced by genetic engineering can be successfully used as an active ingredient in the present invention. Methods for isolation and purification of these proteins are well known to those skilled in the art and are described in detail in papers and books which are readily available.

The proteins are basic glycoproteins having a molecular weight of 70-90 kD that can form complexes by chelating to two ferric iron (Fe³⁺). Since such proteins as transferrin found in blood, lactoferrin found in milk, granulocytes, secretions and ovotransferrin found in avian albumen are throught to be originating from the same ancestor protein because of their structural similarities, they are classified into the class lactoferrin/transferrin family. However, the physiological role of lactoferrin is unclear yet in spite of its wide distribution.

Lactoferrin is chemically unstable and difficult to store without denaturation for more than 2 years by an ordinary storing procedures. To overcome this weak point, it is necessary to avoid excessive purification and to add stabilizers such as carbohydrates, amino acids; the former examples are trehalose and lactose, and the latter sulfur-containing amino acids. In addition, milk proteins like casein and whey protein concentrate are good stabilizers of lactoferrin.

Native and iron-free lactoferrin show antimicrobial activity against certain bacteria, but iron-saturated form does not. Therefore, the antimicrobial activity is dependent on the amounts of iron available for microorganisms in the media, since the activity becomes more potent as the available iron decreases. Thus, the activity is explained by depriving of iron from an environment essential for the growth of microorganisms due to the iron-binding capacity. However, the anti-inflammatory and antimicrobial activity shown in the invention is not attributable to the iron binding capacity, since lactoferrin is a macromolecule and it is highly improbable that it is absorbed from gut as an intact form. Therefore, the mechanism is unclear why orally ingested or topically applied lactoferrin is analgesic and can promote the healing of aphthous lesions.

The present invention is the first instance that lactoferrin shows curative effect on aphthous stomatitis. As mentioned above, the stomatitis is an inflammation so that it produces local edema accompanying with severe pain. Therefore, it is necessary to remove pain for treating it. The present inventors observed that lactoferrin promotes appetite probably due to removal of pain in cats and human having stomatitis. The analgesic activity of lactoferrin is experimentally supported by the fact that neutrophils collected from lactoferrin administered cats are less adhesive to nylon wool column as compared with those derived from control animals. The adhesiveness to nylon wool is directly proportional to the seriousness of inflammation; that is, pain, edema and redness on inflammatory lesion. The decreases in the adhesiveness indicates amelioration in the degree of inflammation.

Lactoferrin also enhanced phagocytic activity of the neutrophils ex vivo, when compared with untreated controls, indicating lactoferrin has both analgesic and immuno-potentiating activities. Furthermore, as Nichols et al. (Pediatr. Res. 21: 563-567, 1987) have shown that lactoferrin is the growth promoter of intestinal crypt cells, the treatment with lactoferrin significantly stimulates mucus healing that is impaired by aphthous stomatitis. From these evidences lactoferrin possesses all of three prerequisites for promoting heal of the disease. The following is a typical experimental results of the present invention.

### Explanation of the experiment:

Among total 16 mongrel cats used as experimental animals, 14 of them were healthy and the rest 2 infected with feline immuno-deficiency virus (FIV) having stomatitis. Bovine milk lactoferrin dissolved in small amounts of water was once daily administered orally to them at a daily dose of 40 mg/kg for 2 weeks, and in the case of FIV-infected cats the administration was continued for additional 2 weeks, because the administration much improved their symptom. During the administration, the blood was withdrawn from carotid vein, and the neutrophils were collected by differential sedimentation according to the method of Nagahata et al. (Vet. Res. Commun. 15: 181-188, 1991) with the use of Ficohl and Conray solution (specific gravity; 1.078). The collected neutrophils were stained by Stenheimer-Marvin method (Commercial name, Uri-Cell; the product of Cambridge Chemicals, Florida, USA) and confirmed they were neutrophils. Then, the suspension of neutrophils was passed through nylon-wool column, and the adsorption rate was calculated according to the following equation; adheion rate = (1- the cells passed through column/the total cells) x 100 (Nagahata et al.: Am. J. Vet. Res. 55: 40-48, 1994)

**Table 1.**

| Effect of lactoferrin on the adhesion rate of neutrophils to nylon wool in healthy cats | | | | |
|---|---|---|---|---|
| cat No. | adhesion rate (%) | | | |
| | before treatment | treatment for 2 weeks | treatment for 4 weeks | 1 month after discontinuation |
| 1 | 40.18 | 15.77 | - | 44.57 |
| 2 | 38.99 | 20.33 | - | - |
| 3 | 40.96 | 31.03 | - | - |
| 4 | 44.32 | 33.28 | 33.39 | - |
| 5 | 52.47 | 43.00 | 37.62 | - |
| 6 | 48.05 | 23.46 | - | - |
| 7 | 42.29 | 31.55 | - | - |
| Mean+SD | 43.89+4.47 | 28.35±8.48* | | |

| | | | | |
|---|---|---|---|---|
| *P<0.01 in Student's t-test. | | | | |

As shown in Table 1, the adhesion of neutrophils to nylon wool column significantly decreased after the treatment for 2 week, suggesting that the treatment analgesic action to the cats.

**Table 2.**

| Effect of lactoferrin on the phagocytic activity of neutrophils in healthy cats | | |
|---|---|---|
| cat No. | phagocytic activity (%) | |
| | before treatment | treatment for 2 weeks |
| 5 | 63.3 | 77.5 |
| 7 | 55.5 | 69.8 |
| 8 | 56.0 | 75.5 |
| 9 | 61.0 | 80.7 |
| 10 | 60.7 | 65.2 |
| 11 | 57,2 | 65.5 |
| 12 | 66.7 | 76.7 |
| mean±SD | 60.1±3.8 | 73.0+5.7* |

| | | |
|---|---|---|
| *P<0.01 in Student's t-test. | | |

Table 2 shows the changes in the phagocytic activity of neutrophils between before and after the treatment (Lima, M.F. and F. Kierszenbaum: J. Immunol, 134: 4176-4183, 1985). It is apparent from the result that lactoferrin treatment significantly potentiates the phagocytic activity of the neutrophils. The changes of phagocytosis rates in cat No. 12 were 66.7% before the treatment, increased to 77.7% 3 days after the initiation of the treatment, to 70.3% on day 7, and to 76.7% on day 14 indicating the activity elevates immediately after the administration.

It is noteworthy that no side effect was seen in the experiment shown above. Oral route is the most preferable upon the administration of but other application routes, for example, topical application with the ointment and oral rinse with the gargle, are also possible. To prepare the pharmaceutical preparation containing protein class lactoferrin family, it is important to avoid protein denaturation. Lactoferrin is compatible with such ordinary pharmaceutical additives as glucose, maltose, sorbitol, dextrin, dextran, starch, microcrystalline cellulose, hydroxypropyl cellulose, carboxymethyl cellulose, talc, either magnesium or calcium stearate, and the granules and the tablets can readily be prepared when mixed with these inactive additives followed by the appropriate mechanical means. The liquid forms also can be made by dissolving it with preservatives.

In order to prepare the foods containing sufficient amounts of lactoferrin, the admixtures with supplemented milk, yogurt, skim milk powder, lactic acid bacteria fermented milk, chocolate, tablet sweets, and powdered beverages are processed by appropriate means. The most important factor is to avoid protein denaturation during processing by not exceeding a temperature of above 60°C. The lactoferrin contents in the processed foods should be more than 150 mg/kg in solid foods and more than 30 mg/200 ml of liquid foods and desirable amounts should be 250 mg/kg food and 50 mg/200 ml liquid. The lactoferrin content in mature raw bovine milk is 60-150 mg/kg.

### Examples

### Example 1-

The gargle containing bovine milk lactoferrin was prepared as follows. Powdered bovine lactoferrin 5 g was mixed with trehalose 0.1 g, aluminum potassium sulfate 3.0 g, mentha water (Pharmacopoea Japonica) 50 ml, and the mixture was filled up to 1 liter with purified water (Pharmacopoea Japonica).

### Example 2-

Oral ointment was prepared as follows. Micronized sodium salt of carboxymethylcellose 50 g was mixed thoroughly with Hydrocarbon Gel (Japanese Standards of Pharmaceutical Ingredients, pp1254, 1991) 100 g. To this mixture 2 drops of vanilla essence was added. Crude bovine lactoferrin consisting of 51% of lactoferrin and 11 % of lactoperoxidase was mixed with the mixture, 100 g, prepared above.

### Example 3

Male cat, 4.5 years old, infected with FIV, was treated with bovine lactoferrin. The animal had stomatitis concomitant with poor appetite one year ago slavering heavily. The symptoms were marked granulomatous hyperplasia and erosion of ulcerous gingiva, and pain. Bovine milk lactoferrin was orally administered at a daily dose of 40 mg/kg, and the symptoms were much improved and the appetite restored normal on day 7. Neutrophil adhesion to nylon wool column gradually decreased from 43.57% before the treatment to 44.75% on week 2 and to 18.53% on week 4.

### Example 4-

Female cat, 1.5 years old, had stomatitis a few months ago and erosion on tongue with bleeding and angular stomatitis with granuloma were observed. Bovine milk lactoferrin was orally administered at a daily dose of 40 mg/kg. The treatment much ameliorated these symptoms on week 2, although smaller granuloma still remained. The phagocytic activity of neutrophils much enhanced by the treatment; the rate of phagocytosis increased from 58.3% before treatment to 92.7% on week 2 of treatment.

### Example 5-

A man, 33 years old, have had marginal periodontitis characterized by gingival redness, swelling and bleeding since January 4, 1994. Especially, these symptoms were severe at the locus of left mandible molar. The focal infection was treated by drainage and antibiotic, the dental calculus was removed and guidance of brushing was made by the dentist. However, the redness and swelling at the locus of left mandible molar were still present. He was orally treated with lactoferrin 3 times per day at a daily dose of 360 mg (120 mg x 3) concomitant with topical application of minocycline ointment. The swelling began to disappeared on day 3, and the symptoms disappeared on day 7. The treatment showed no side effect and the efficacy was excellent in this refractory case.

### Example 6-

A woman, 55 years old, had stomatitis, and she was treated with oral lactoferrin therapy. She received daily 360 mg of lactoferrin at 3 equally divided dose. She had a circular ulcer of 7 mm in diameter a several years ago at the locus of right maxilla dogtooth. After the ulcer disappeared, it recurred 2-3 month intervals. Once the ulcer relapsed, the patient was suffered severe pain for 5-7 days. The effect of glucocorticoid ointment was transient, and lactoferrin was used instead of it. The pain ameliored on day 3, and the ulcer completely healed on day 7. The patient felt no objective symptoms. There was no side effect and the efficacy was excellent.

### Example 7-

A woman, 48 years old, was suffered from marginal periodontitis complicated with ulcerative gingivitis. She felt severe pain at the loci of both maxilla dogteeth and a premolar tooth with redness and erosion. She was treated by removing dental calculus. The focal infection was removed by brushing guidance but the symptoms little disappeared. She was treated by Chinese herbal medicines 9 months before, but it was no effect. Therefore, oral lactoferrin therapy was initiated instead the herbal medicines. The erosion disappeared by the treatment, but the redness still remained, although it became much smaller. The pain also disappeared by the treatment. The efficacy of this case was judged as good.

### Example 8-

A woman, 71 years old, had periodontal abscess at the locus of right maxilla middle incisor since January 15, 1995. The treatment was initiated from January 17 1995; redness and swelling were noted at the inflammatory locus, and drainage was made from marginal gingiva simultaneous oral lactoferrin therapy, 360 mg/day (120 mg x 3 per day). The redness and swelling were still present on day 2, but the secretion changed from pus-like to serous one. Therefore, amoxicillin was orally administered for 3 days with lactoferrin. On day 7 the symptoms mentioned above disappeared. This case also showed no side effect and lactoferrin synergistically exerted antimicrobial activity against pathogens infected in gingiva.

Further variations and modifications of the foregoing will be apparent to those skilled in the art and such variations and modifications are attended to be encompassed by the claims that are appended hereto.

## Claims

1. A pharmaceutical composition for treatment and/or prevention of oral inflammation such as aphthous stomatitis and gingivitis comprising (a) a protein belonging to the class of transferrin/lactoferrin family and (b) a pharmaceutically acceptable carrier.

2. The composition according to Claim 1, where said protein is selected from lactoferrin, transferrin and ovo transferrin or the protein belongs to the mammalian transferrin/lactoferrin family independent of its iron saturation including from iron-free apo-lactoferrin to 100% iron-saturated holo-lactoferrin.

3. The composition according to Claim 1, where said aphthous stomatitis is induced by chronic administration of anticancer chemotherapies.

4. The composition according to Claim 1, where said aphthous stomatitis is complicated with autoimmune diseases, such as Bechet disease, SLE and others, or with periodontal diseases of the oral cavity.

5. A composition according to Claim 1 in the form of a tablet, a hard gelatin capsule, an ointment, a cream or a gargle.

6. Use of a protein belonging to the class of transferrin/lactoferrin family in the preparation of a medicament for the treatment of oral inflammation such as aphthous stomatitis and gingivitis.

7. Use according to Claim 5, wherein said protein is present in the medicament in an amount to give a dosage of 0.1 to 250 mg/kg/day, preferably 0.1 to 100 mg/kg/day, more preferably 1 to 50 mg/kg/day.

8. A food product which comprises food and an amount of a pharmaceutical composition effective to prevent and/or treat oral inflammation such as aphthous stomatitis and gingivitis, said pharmaceutical composition comprising (a) protein belonging to the class of transferrin/lactoferrin family, and (b) optionally a pharmaceutical acceptable carrier.

9. The food product according to Claim 8, wherein said lactoferrin is present in an amount over 1 mg/kg body weight preferably over 5 mg/kg body weight and said food product is solid.

10. The food product according to Claim 8, wherein said lactoferrin is present in an amount over 30 mg/ml, preferably over 50 mg/ml and said food product is liquid.
